# EUROPEAN PATENT APPLICATION

(11) **EP 4 821 437 A1**
(43) Date of publication of application: **14.10.2026**
(21) Application number: 25169760.3
(22) Date of filing: 10.04.2025
(51) Int. Cl.: A61B 5/346, A61B 5/00, A61B 5/024

(54) **PREDICTING AND CLASSIFYING PARKINSON'S DISEASE SEVERITY AND PROGRESSION FROM ECG**

(71) Applicant: Koninklijke Philips N.V., 5656 AE Eindhoven (NL)
(72) Inventor: AN, Junmo, Eindhoven (NL)
(74) Representative: Philips Intellectual Property & Standards

(57) **Abstract**

A system for classifying and projecting neurological disease severity based on electrocardiograph (ECG) includes a controller comprising a memory that stores instructions and a processor that executes the instructions. When executed by the processor, the instructions cause the system to: obtain initial ECG data; detect characteristics indicative of a neurological disease from the initial ECG data; classify severity of the neurological disease based on the detected characteristics from the initial ECG data; and predict a future severity of the neurological disease based on the classified severity.

## Description

### BACKGROUND OF THE INVENTION

Electrocardiograms (ECGs) are essential diagnostic tools that provide a detailed view of the heart's electrical activity. A standard 12-lead ECG records this activity from 12 different leads, offering a comprehensive snapshot of cardiac function over a typical 10-second interval. This tool is indispensable in clinical practice for diagnosing a wide range of cardiac conditions, from arrhythmias to myocardial infarction, due to its accuracy, reliability, and ease of use. While primarily used for cardiac assessments, emerging research highlights the potential of ECGs in detecting and monitoring non-cardiac conditions, including neurodegenerative and other neurological diseases.

Parkinson's Disease (PD) is a chronic, progressive neurodegenerative disorder characterized by both motor symptoms (e.g., tremor, bradykinesia, rigidity) and non-motor symptoms (e.g., autonomic dysfunction, sleep disturbances, and mood disorders). These symptoms collectively impose a significant burden on patients, affecting their quality of life and necessitating frequent monitoring and treatment. Current PD assessment methods primarily rely on subjective, infrequent, manual clinical observations, such as the Hoehn and Yahr (H&Y) scale or the Unified Parkinson's Disease Rating Scale (UPDRS), which may lack consistency and fail to capture subtle changes over time. Current clinical variations vary depending on clinician expertise. These evaluations are influenced by inter-observer variability. Imaging techniques, such as Dopamine Transporter Scan (DaTscan), Single Photon Emission Computed Tomography (SPECT), Positron Emission Tomography (PET) scans, or others, are expensive, resource-intensive, and not widely available for routine monitoring. As a result, regular and objective monitoring of disease progression is currently inefficient and challenging with existing methods.

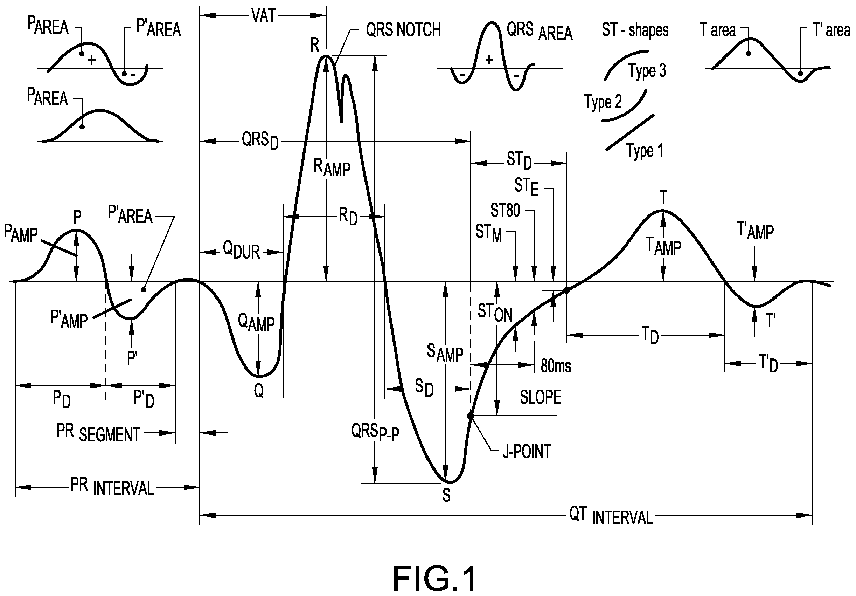

Fig. 1 illustrates conventional ECG morphology measurements, likely depicting a standard ECG waveform with key morphological features, including amplitudes, intervals, and areas used in clinical assessment. These measurements provide insights into the heart's electrical activity and aid in diagnosing various cardiac conditions.

Recent studies have demonstrated that autonomic dysfunction, a common non-motor manifestation of Parkinson's Disease (PD), can be detected through ECG-derived parameters. For example, alterations in heart-rate variability (HRV), QT interval prolongation, and other electrophysiological markers have been linked to PD. Furthermore, recent research highlights the effectiveness of machine learning and deep learning models in distinguishing PD-related ECG patterns from those of healthy individuals and those with other cardiac abnormalities. This growing body of evidence emphasizes the significant, yet underexplored, potential of ECGs in enabling automated and scalable PD diagnosis and monitoring. However, current research and methodologies remain limited in their ability to classify PD severity and systematically assess disease progression over time using ECG data. Despite potential evidence linking autonomic dysfunction in Parkinson's Disease (PD) with ECG abnormalities, this data remains significantly underexplored for severity classification and progression assessment applications.

### SUMMARY OF THE INVENTION

According to an aspect of the present disclosure, a system for classifying and projecting neurological disease severity based on electrocardiograph (ECG) includes a controller comprising a memory that stores instructions and a processor that executes the instructions. When executed by the processor, the instructions cause the system to: obtain initial ECG data; detect characteristics indicative of a neurological disease from the initial ECG data; classify severity of the neurological disease based on the detected characteristics from the initial ECG data; and predict a future severity of the neurological disease based on the classified severity.

According to another aspect of the present disclosure, a method for training an artificial intelligence model to classify neurological disease severity based on electrocardiograph (ECG) includes: obtaining ECG data from a plurality of patients diagnosed with a neurological disease across a spectrum of stages, and from a plurality of individuals without the neurological disease; labeling the ECG data according to the corresponding severity stage for patients and as normal for individuals without the neurological disease; extracting ECG features associated with characteristics of the neurological disease from the labeled ECG data; processing the extracted ECG features and the ECG data to identify patterns related to disease severity; and developing a classifier model as the artificial intelligence model to classify the ECG data based on the ECG features and the identified patterns.

According to another aspect of the present disclosure, a method for classifying and projecting neurological disease severity based on electrocardiograph (ECG) is performed using a controller comprising a memory that stores instructions and a processor that executes the instructions. The method includes the processor executing the instructions to implement the method. The method comprises obtaining initial ECG data; detecting characteristics indicative of a neurological disease from the initial ECG data; classifying severity of the neurological disease based on the detected characteristics from the initial ECG data; and predicting a future severity of the neurological disease based on the classified severity.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 illustrates conventional ECG morphology measurements.
Fig. 2 illustrates a system for predicting and classifying Parkinson's disease severity and progression from ECG, in accordance with a representative embodiment.
Fig. 3 illustrates a progression of Parkinson's disease severity, in accordance with a representative embodiment.
Fig. 4 illustrates representative 12-lead ECGs for a healthy individual and two different individuals with different severities of Parkinson's disease in predicting and classifying Parkinson's disease severity and progression from ECG, in accordance with a representative embodiment.
Fig. 5 illustrates a method for predicting and classifying Parkinson's disease severity and progression from ECG, in accordance with a representative embodiment.
Fig. 6 illustrates a method for predicting and classifying Parkinson's disease severity and progression from ECG, in accordance with a representative embodiment.
Fig. 7 illustrates a method for predicting and classifying Parkinson's disease severity and progression from ECG, in accordance with a representative embodiment.
Fig. 8 illustrates a method for predicting and classifying Parkinson's disease severity and progression from ECG, in accordance with a representative embodiment.
Fig. 9 illustrates progressions and progressions for predicting and classifying Parkinson's disease severity and progression from ECG, in accordance with a representative embodiment.
Fig. 10 illustrates a deep learning (DL) model for predicting and classifying Parkinson's disease severity and progression from ECG, in accordance with a representative embodiment.
Fig. 11 illustrates a computer system, on which a method for predicting and classifying Parkinson's disease severity and progression from ECG is implemented, in accordance with another representative embodiment.

### DETAILED DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a,' 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, the severity of Parkinson's disease and other neurological diseases may be classified and tracked using multi-lead electrocardiograms (ECGs). The methods and system described herein provide an objective, continuous, and easily accessible approach to PD monitoring. By leveraging machine learning or deep learning algorithms applied to ECG data, the methods and system can classify PD severity into different stages and monitor disease progression over time, such as daily, monthly, or yearly. The methods and system facilitate improved disease management, support the development of personalized treatment strategies, and enhance the design and execution of clinical trials. By addressing the limitations of traditional clinical assessments, this method fulfills a critical need for frequent, reliable, and objective PD monitoring.

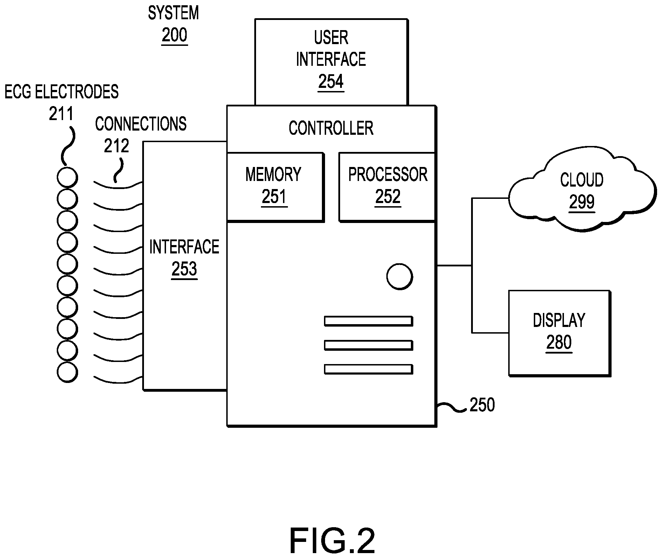

Fig. 2 illustrates a system 200 for predicting and classifying Parkinson's disease severity and progression from ECG, in accordance with a representative embodiment.

The system 200 in Fig. 2 is a system for predicting and classifying Parkinson's disease severity and progression from ECG and includes components that may be provided together or that may be distributed. The system 200 includes a controller 250, a display 280, a cloud 299, and ECG electrodes 211 connected by connections 212 to an interface 253 of the controller 250. The controller 250 includes a memory 251 that stores instructions, a processor 252 that executes the instructions, the interface 253 for the ECG electrodes 211, and a user interface 254.

ECG electrodes 211 are sensors placed on the body to detect electrical signals. Leads are the electrical signals or perspectives captured from the ECG electrodes 211. The leads are transmitted to the interface 253 via connections 212. An example of the connections 212 is cables. While the system 200 includes ten ECG electrodes 211 and connections 212 for a 12-lead ECG configuration, the teachings herein are applicable to both single-lead and multi-lead ECG signals, including variations such as 2-, 3-, 4-, 5-, 12-, 15-, 16-, 22-, 80-lead, and so on. The ECG electrodes 211 may be used to provide input data such as 12-lead ECG recordings from patients suspected or diagnosed with Parkinson's disease.

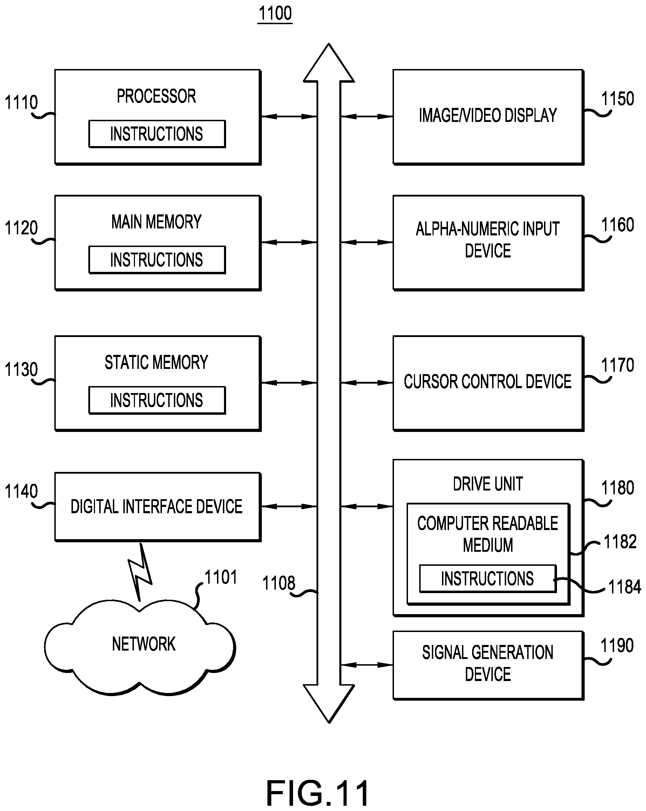

The controller 250 includes at least the memory 251 that stores instructions and the processor 252 that executes the instructions. A computer that can be used to implement the controller 250 is depicted in Fig. 11, though a controller 250 may include more or fewer elements than depicted in Fig. 2 or Fig. 11. In some embodiments, multiple different elements of the system 200 in Fig. 2 may include a controller such as the controller 250. For example, the display 280 may also include an embedded controller. The user interface 254 may include a display separate from the display 280, as well as input mechanisms such as buttons, keys, knobs, a mouse, a microphone, a touch screen, a trackpad, a stylus, voice recognition systems, gesture controls, or other interactive elements by which a user can provide inquiries or instructions to the system 200.

The user interface 254 and/or the display 280 may provide a dashboard for clinicians to visualize results, track progression, and receive alerts on significant changes.

The display 280 may be connected to the controller 250 via a local wired interface such as an Ethernet cable or via a local wireless interface such as a Wi-Fi connection. The display 280 may be interfaced with other user input devices by which users can input instructions, including mouses, keyboards, thumbwheels and so on. The display 280 may be a monitor such as a computer monitor, a display on a mobile device, an augmented reality display, a television, an electronic whiteboard, or another screen configured to display electronic imagery. The display 280 may also include one or more input interface(s) that may connect to other elements or components, as well as an interactive touch screen configured to display prompts to users and collect touch input from users.

The controller 250 may include additional interfaces, such as a first additional interface, a second additional interface, a third additional interface, and a fourth additional interface. One or more of the interface 253, the user interface 254 or any additional interface may include ports, disk drives, wireless antennas, or other types of receiver circuitry that connect the controller 250 to other electronic elements. One or more of the interface 253 or any additional interface may also include user interfaces such as buttons, keys, a mouse, a microphone, a speaker, a display separate from the display 280, or other elements that users can use to interact with the controller 250 such as to enter instructions and receive output.

The controller 250 may perform some of the operations described herein directly and may implement other operations described herein indirectly. For example, the controller 250 may indirectly control operations such as by generating and transmitting content to be displayed on the display 280. The controller 250 may directly control other operations such as logical operations performed by the processor 252 executing instructions from the memory 251 based on input received from electronic elements such as the ECG electrodes 211 as well as input received users via the user interface 254 or another user interface. Accordingly, the processes implemented by the controller 250 when the processor 252 executes instructions from the memory 251 may include steps not directly performed by the controller 250.

The controller 250 obtains ECG data from the ECG electrodes via the connections 212 and the interface 253.

The cloud 299 is representative of a network cloud, and may be implemented using remote servers such as in data centers. The cloud 299 may be used to implement a service that provides analyses for multiple patients with Parkinson's disease, potentially accommodating large numbers such as 10,000, 20,000, or even more. The cloud 299 may be used to train artificial intelligence models and/or to implement artificial intelligence models in the manners described herein. While program that implements some or all of the teachings herein may be implemented in or using the cloud 299, software used to implement these teachings may also or alternatively be deployed at a network edge on portable ECG devices or via electrocardiographs.

Using the system 200, the diagnostic power of 12-lead ECGs is leveraged to classify PD severity and to monitor disease progression over time such as daily, monthly, and yearly intervals. Advanced machine learning or deep learning algorithms may be applied to ECG data by the controller 250 and/or in the cloud 299 so that the system 200 identifies subtle electrophysiological changes linked to PD progression, offering a non-invasive, cost-effective, and widely accessible solution for disease management.

The system 200 provides accurate severity classification of PD into Hoehn and Yahr stages, enabling healthcare providers to better understand disease severity and tailor treatment plans accordingly. The continuous monitoring of PD progression over various timeframes offers a dynamic view of disease evolution that is essential for personalized care. Utilization of widely available 12-lead ECGs ensures this system is scalable and accessible to the point that the system 200 can be easily implemented in clinical settings and potentially adapted for home monitoring. The objective assessment offered by leveraging artificial intelligence models and the cloud 299 offers objective assessments which eliminate reliance on subjective clinical evaluations, providing consistent and reproducible results. The system 200 allows integration with existing workflows and is designed to complement current clinical practices so that the system 200 is seamlessly integrated into electronic health records (EHRs) and used alongside other diagnostic tools.

In some embodiments, the system 200 may be implemented using a mobile electronic device. For instance. The controller 250 may be implemented in a mobile electronic device that is configured to electronically communicate with or connect to the ECG electrodes 211 and connections 212, as well as the display 280.

In Fig. 2, either or both of the user interface 254 and the display 280 may serve as a User Interface: A user-friendly interface for clinicians to visualize and interpret the results.

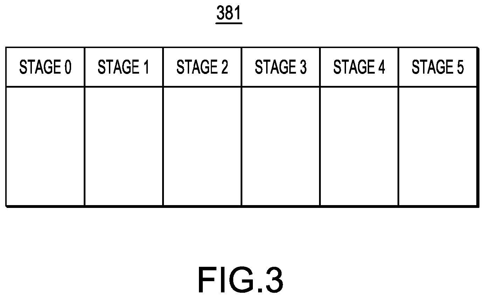

Fig. 3 illustrates a progression of Parkinson's disease severity, in accordance with a representative embodiment.

The progression in Fig. 3 is shown on a user interface 381, such as by a user interface on the display 280 in Fig. 2. The progression shows six stages which may be representative of Parkinson's disease. While the Hoehn and Yahr scale has five stages (1 to 5) and Stage 0 indicates no signs of disease, Stages 1 and 2 each may have two parts. A first part of the first stage (Stage 1) may reflect unilateral involvement only with no significant disability, so tremor or other movement symptoms on one side of the body only. A second part of the first stage (Stage 1.5) may reflect unilateral involvement with axial symptoms, so difficulty with posture or gait. A first part of the second stage (Stage 2) may reflect bilateral involvement and no impairment of balance, or mild bilateral involvement with recovery on the retropulsion test. A second part of the second stage (Stage 2.5) may reflect mild bilateral disease with recovery on a retropulsion test. The third stage (Stage 3) may reflect mild to moderate involvement and some postural instability while the patient is still physically independent. The fourth stage (Stage 4) may reflect a severe disability while the patient is still able to walk or stand unassisted but is increasingly dependent. The fifth stage (Stage 5) may reflect the patient being wheelchair-bound or bedridden unless assisted. The six stages in Fig. 3 reflect a similar progression of differentiable stages detectable and traceable using the teachings herein. Several scales can also be used to predict or classify various aspects of the participants' health, motor function, balance, gait, and quality of life:
- Hoehn and Yahr Scale: Used to assess the stage of Parkinson's disease. Ranges from 1 to 5. In some cases, intermediate stages such as 1.5 or 2.5 may be used to better capture more nuanced stages of disease progression. Higher scores indicate more severe motor symptoms. Relative to the Hoehn and Yahr Scale, Stage 0 of Fig. 3 may indicate no signs of disease, while the next five stages may correspond to the five stages of the Hoehn and Yahr scale. The display 280 or user interface 254 may illustrate Stage 0 and the five affirmative stages of Hoehn and Yahr scale with icons indicating a progression for Parkinson's disease, such as with a human figure standing fully upright at Stage 0 when the body is unaffected; a human figure standing upright relatively unaffected at Stage 1 when only one side of the body is affected; a human figure slightly bent over at Stage 2 when symptoms affect both sides of the body; a human figure bent over at Stage 3 when balance and stability become affected; a human figure using a walker at Stage 4 when symptoms increase; and a human figure in a wheelchair at Stage 5 when assistance is required for everyday activities.
- Functional Ambulation Category (FAC): Evaluates the walking ability of patients. Ranges from 0 to 5. Higher scores indicate better walking ability.
- Rankin Scale (Modified Rankin Scale, mRS): Measures the degree of disability or dependence in daily activities. Ranges from 0 to 5. Higher scores indicate greater disability.
- Geriatric Depression Scale (GDS): Assesses depression in elderly patients. Ranges from 0 to 4. Higher scores indicate more severe depression.
- Mini-Mental State Examination (MMSE): Evaluates cognitive function. Ranges from 0 to 30. Scores ≥24 are generally considered normal cognitive function.
- Barthel Index: Measures functional independence in activities of daily living. Ranges from 0 to 100. Higher scores indicate greater independence in activities of daily living.
- Tinetti's Performance-Oriented Mobility Assessment (POMA): Assesses balance and gait. POMA Balance: Ranges from 0 to 16; POMA Gait: Ranges from 0 to 12; POMA Total: Ranges from 0 to 28. Higher scores indicate better balance and gait.
- Short Physical Performance Battery (SPPB): Evaluates lower extremity function. Ranges from 0 to 12. Higher scores indicate better lower extremity function.
- 6-Minute Walking Test: Measures the distance walked in six minutes as an indicator of functional capacity. No fixed range, but higher distances indicate better functional capacity.
- Timed Up and Go Test (TUG): Assesses mobility and fall risk. No fixed range, but shorter times indicate better mobility and lower fall risk.
- 12-Item Short-Form Health Survey (SF-12): Evaluates health-related quality of life. Physical Component Score (PCS-12): Ranges from 0 to 100; Mental Component Score (MCS-12): Ranges from 0 to 100. Higher scores indicate better health-related quality of life.
- Falls Efficacy Scale-International (FES-I): Measures fear of falling. Ranges from 16 to 64. Higher scores indicate greater fear of falling.
- Unified Parkinson Disease Rating Scale - III (UPDRS-III): Assesses motor symptoms of Parkinson's disease. Ranges from 0 to 108. Higher scores indicate more severe motor symptoms.
- Others.

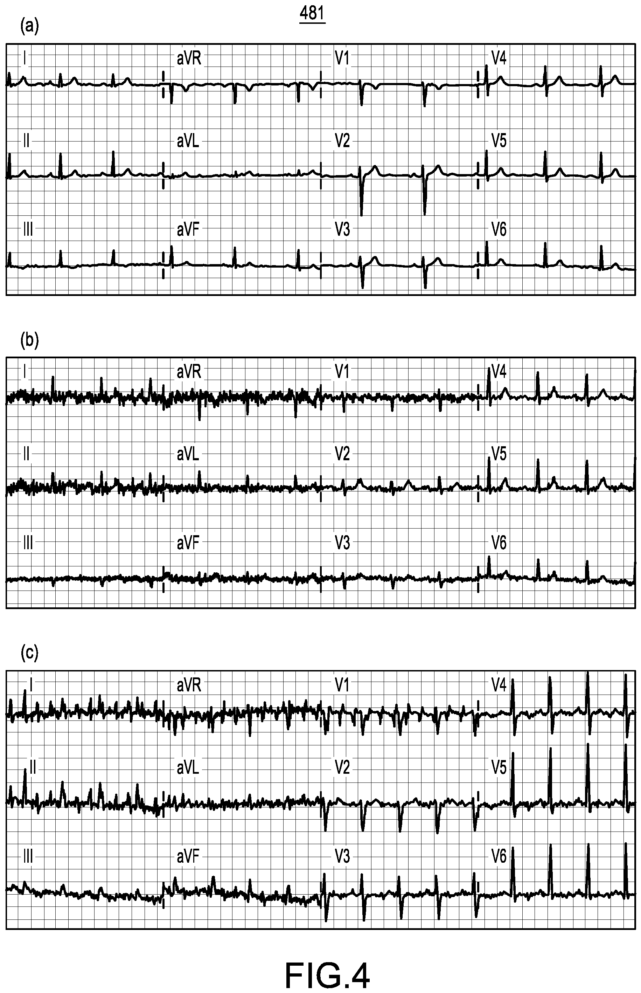

Fig. 4 illustrates representative 12-lead ECGs for a healthy individual and two different individuals with different severities of Parkinson's disease in predicting and classifying Parkinson's disease severity and progression from ECG, in accordance with a representative embodiment.

Fig. 4 shows a representative 12-lead ECGs illustrating (a) a healthy adult and (b, c) individuals with different severities of Parkinson's Disease (PD).

Fig. 4 shows a user interface 481 on a display such as the display 280. The user interface 481 shows representative 12-lead ECG recordings: (a) from a healthy adult and (b, c) from individuals with varying severities of Parkinson's Disease (PD).

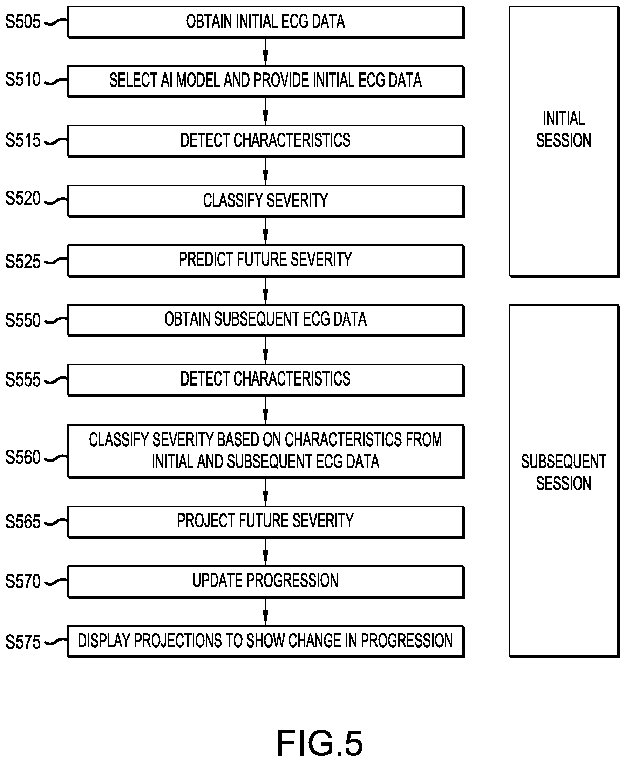

Fig. 5 illustrates a method for predicting and classifying Parkinson's disease severity and progression from ECG, in accordance with a representative embodiment.

The method of Fig. 5 may be performed by the system 200 including the controller 250. The method of Fig. 5 is broken up into two sessions including an initial session from S505 to S525 and a subsequent session from S550 to S575.

At S505, initial ECG data is obtained in the initial session.

At S510, an artificial intelligence model is selected from a plurality of available artificial intelligence models. The initial ECG data is provided to the selected artificial intelligence model. The controller 250 is configured to provide the initial ECG data as input to the selected artificial intelligence model to detect the characteristics indicative of the neurological disease. Example artificial intelligence models include at least one of a classification algorithm for classifying severity in different stages or scores, or a regression model for tracking the progression of a neurological disease in a patient over time. The controller 250 may be configured to select an artificial intelligence model from among a plurality of available artificial intelligence models based on predefined selection criteria; and apply the selected artificial intelligence model to the initial ECG data to detect the characteristics of the neurological disease. Selection criteria may be predefined based on the particular neurological disease to be detected, the type of patient, and/or preferences of the clinician responsible for the ECG session. A clinician may input the selection criteria via the user interface 254, for example.

Several different types of machine learning models may be made available for the selection at S510 and the subsequent application to ECG data. One example type is a classification algorithm. Examples of classification algorithms include support vector machines, random forests, or deep learning networks. Deep learning models may include convolutional neural networks (CNNs) or recurrent neural networks (RNNs), for end-to-end deep learning from raw ECG signals or segmented features. Another example type is a regression model. Examples of regression models include linear regression, polynomial regression, gradient boosting models (e.g., XGBoost, LightGBM, CatBoost), random forest regression, RNNs, Long Short-Term Memory Networks (LSTMs) and Gated Recurrent Units (GRUs), Temporal Convolutional Networks (TCNs), and transformer-based models (e.g., Time-Series Transformers, BERT, etc.), among others, to estimate progression trajectories over daily, monthly, or yearly intervals.

At S515, characteristics are detected by the selected artificial intelligence model from the initial ECG data. The controller 250 is configured to detect characteristics indicative of a neurological disease from the initial ECG data from S505. The detected characteristics may comprise electrophysiological markers such as at least one of heart-rate variability (HRV) metrics, QT interval, QRS duration, and frequency-domain features.

At S520, severity of Parkinson's disease or another neurological disease is classified based on the detected characteristics. The controller 250 is configured to classify severity of the neurological disease based on the detected characteristics from the initial ECG data from S515. The controller 250 may be configured to generate and output a severity stage of the neurological disease along with a confidence score for the severity stage. Examples of severity stages are shown in and described with respect to Fig. 3. Confidence scores may be provided to demonstrate a statistical confidence in a severity classification for the severity stage of the patient. An example confidence score may be 80%, or a window of 80-90%. The controller 250 may implement a severity classification module as a machine learning or deep learning model for classifying the severity of Parkinson's disease into five stages based on the Hoehn and Yahr scale.

At S525, future severity of the Parkinson's disease or another neurological disease, such as Alzheimer's disease, stroke, or others, is predicted based on the initial ECG data, the characteristics detected at S515, and the severity classified at S520. The controller 250 is configured to predict a future severity of the neurological disease based on the classified severity from S520.

The subsequent session begins at S550 by obtaining subsequent ECG data in the subsequent session. The subsequent session is later than the initial session, such as a week later or a month later. The controller 250 or another instance of the controller 250 is configured to obtain subsequent ECG data of the same patient as in S505 through S525.

At *S555,* characteristics are detected by the same artificial intelligence model from S510, but now from the subsequent ECG data. The controller 250 or another instance of the controller 250 is configured to detect characteristics of the neurological disease from the subsequent ECG data from S550.

At S560, severity of Parkinson's disease or another neurological disease is classified based on the detected characteristics from the initial ECG data obtained at S505 and from the subsequent ECG data obtained at S550. The controller 250 or another instance of the controller 250 is configured to classify severity of the neurological disease based on the detected characteristics from the subsequent ECG data from S555. As noted with respect to S520, the controller 250 may be configured to generate and output a severity stage of the neurological disease along with a confidence score for the severity stage.

At S565, future severity of the Parkinson's disease or another neurological disease is predicted based on the subsequent ECG data, the characteristics detected at S555, and the severity classified at S560. The future severity predicted at S565 may also be predicted based on the initial ECG data, the characteristics detected at S515, and the severity classified at S520 together with the subsequent ECG data, and detected characteristics and classified severity from the subsequent session. The controller 250 or another instance of the controller 250 is configured to update the predicted future severity of the neurological disease from S525 from both the initial ECG data and subsequent ECG data.

At S570, progression of the Parkinson's disease or another neurological disease is updated based on the new information from the subsequent session. The controller 250 or another instance of the controller 250 is configured to update the disease progression based on the classified severity from both the initial ECG data and subsequent ECG data. The controller 250 or another instance of the controller 250 is configured to track a progression of the neurological disease for a patient over time by analyzing the classified severity from the initial ECG data and at least one previous ECG dataset. The progression may be tracked and updated over time such as weekly, monthly or seasonally, and projections from previous sessions may be compared to an updated progression to see if a prognosis is worse or better than previously projected. The controller 250 may implement a progression assessment module as a regression model for assessing the progression of Parkinson's disease over different time intervals (daily, monthly, yearly).

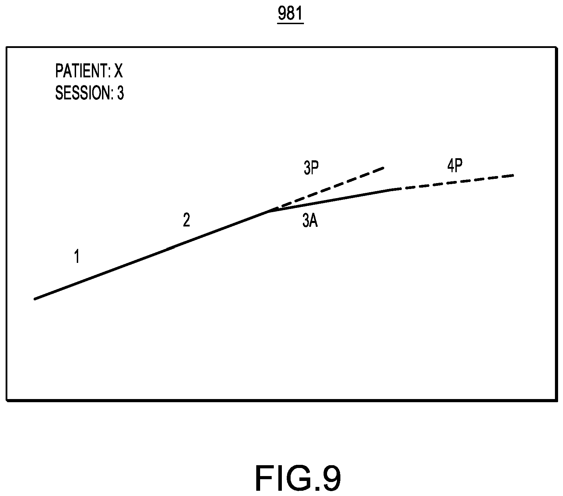

At S575, projections of the future severity are displayed such as on the display 280 to show change in progression between the initial session and the subsequent session. An example of a display of projections of future severity is shown in Fig. 9. The display 280 is configured to visually present the predicted progression of disease severity over multiple ECG datasets. The output displayed at S575 may include progression metrics, including rate of change and projected future severity. For monitoring Parkinson's disease progression, the H&Y stages automatically classified by the model may be displayed using bar graphs or other types of visualizations, showing changes over daily, monthly, or yearly intervals.

The method of Fig. 5 may involve two or more modes. The process for classifying Parkinson's disease severity into the five Hoehn and Yahr (H&Y) stages and assessing progression in patients involves two operational modes: detection mode and serial comparison mode. In detection mode, the method includes selecting the most appropriate model or models, whether a feature-based machine learning model, an end-to-end deep learning model, or an integrated hybrid approach. The selected model classifies Parkinson's disease into the five H&Y stages (1 to 5) with the ability to provide finer granularity by identifying intermediate stages, rounded to one decimal place. The detection mode also includes functionality for distinguishing Parkinson's disease patients from normal or healthy individuals. In serial comparison mode, the focus is on tracking the progression of known Parkinson's disease cases over time. Monitoring intervals can be adjusted to daily, weekly, monthly, or yearly assessments based on clinical needs. The serial comparison mode may use regression models, developed through machine learning, deep learning, or a hybrid approach, to predict and quantify changes in disease severity over time. The trained models are designed for versatility and can be deployed across a variety of platforms such as on-device systems such as smartphones, tablets, portable ECG devices, and electrocardiographs, as well as remote environments such as cloud-based or server-based systems, ensuring accessibility and scalability for diverse clinical settings.

The controller 250 may be configured to initiate a first mode to detect the characteristics of the neurological disease, including selecting the artificial intelligence model to apply the selected artificial intelligence model to the initial ECG data. The controller 250 may also be configured to initiate a second mode to track progression of known cases of the neurological disease over time across a plurality of patients. After each session, the controller 250 may be configured to store the detected characteristics, classified severity, and predicted progression of the neurological disease in a secured cloud-based repository such as the cloud 299, or a server, or an electrocardiograph device.

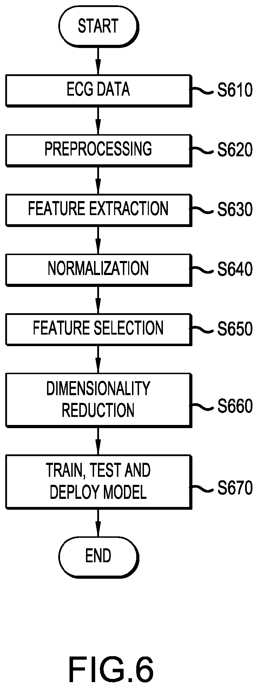

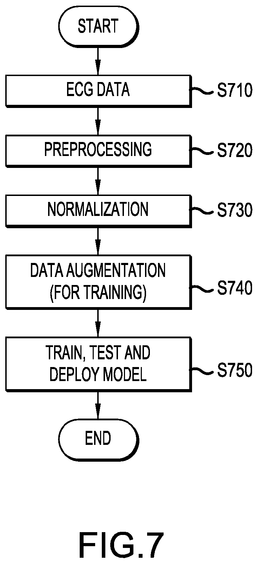

To classify and assess severity in Parkinson's disease using multi-lead ECG data, two main approaches can be utilized: feature-based machine learning and end-to-end deep learning, both of which are effective. A feature-based machine learning (ML) model is the subject of Fig. 6, and an end-to-end deep learning approach is the subject of Fig. 7.

Fig. 6 illustrates a method for predicting and classifying Parkinson's disease severity and progression from ECG, in accordance with a representative embodiment.

Fig. 6 shows a flowchart illustrating the procedure for preparing data to train, test, and deploy a feature-based machine learning (ML) model for reliably classifying Parkinson's Disease (PD) severity into the five Hoehn and Yahr (H&Y) stages and monitoring disease progression in patients using multi-lead ECG data. Multi-lead ECG data may be incorporated as input to capture spatial and temporal patterns across all multiple leads for improved classification accuracy.

The method of Fig. 6 may be performed by the system 200 including the controller 250.

At S610, ECG data is obtained. ECG data may be acquired by the system 200. While the system 200 includes a 12-lead ECG, configurations ranging from single-lead to 2-, 3-, 4-, 5-, 12-, 15-, 16-, 22-, 80-lead, and beyond may also be utilized. For AI training described herein for Figs. 6, 7 and 8, ECG data may comprise 12-lead ECG data from Parkinson's disease patients across all Hoehn and Yahr (H&Y) stages 1 to 5 and from control subjects, including healthy adults without Parkinson's disease or other neurological conditions. The underlying ECG data should consist of ECG signals recorded under standardized conditions to minimize variability, ensuring consistency in lead placement, signal duration, and sampling frequency. The dataset should be diverse and representative, covering a wide range of age groups, genders, and ethnicities to improve generalizability.

At S620, the ECG data is preprocessed. A preprocessing module of software executed by the controller 250 may comprise one or more algorithms for cleaning and preprocessing ECG signals to remove unwanted artifacts and noise such as power line interference and baseline wander, and muscle noise, and to extract relevant features at S630. Preprocessing steps at S620 may include noise removal and signal normalization. Signal filtering techniques, such as low-pass, high-pass, or band-pass filters, may be applied to eliminate frequency-specific noise if needed. Advanced methods such as wavelet transform or empirical mode decomposition (EMD) may be used to address complex noise patterns while preserving signal integrity if needed. Signal irregularities such as missing data or lead-off errors, using interpolation or other restoration techniques may be detected and corrected.

At S630, features are extracted from the preprocessed ECG data. The process involves extracting various features associated with autonomic dysfunction and electrophysiological abnormalities from ECG data, including temporal intervals such as RR intervals, PR intervals, QRS duration, QT intervals, and corrected QT intervals (QTc). Extracted features may also include heart-rate variability (HRV) metrics, including time-domain measures (e.g., SDNN, RMSSD), frequency-domain measures (e.g., LF/HF ratio), and nonlinear metrics (e.g., Poincaré plots and entropy measures). Additional features include amplitude characteristics such as P-wave amplitude, QRS amplitude, T-wave amplitude, and ST-segment deviations; morphological features capturing the shape and slope of P, QRS, and T waves, as well as lead-specific ECG patterns. Advanced signal processing techniques such as probabilistic symbolic pattern recognition (PSPR) may be applied to identify patterns in ECG signals that correlate with Parkinson's disease severity. Other advanced signal processing techniques may be applied such as wavelet transform or empirical mode decomposition (EMD) for time-frequency analysis and feature extraction from non-stationary signals. Principal component analysis (PCA) or independent component analysis (ICA) may be applied for dimensionality reduction and identification of lead-specific contributions. These features collectively provide insights into cardiac abnormalities or conditions commonly associated with Parkinson's disease. Lead-specific and multi-lead derived features may be incorporated to capture the spatial distribution of electrical activity across different perspectives of the heart. Dynamic features may be evaluated by analyzing ECG signals over longer timeframes or during specific tasks (e.g., deep breathing or orthostatic tests) to assess autonomic response. The extracted features may be validated against clinical markers of Parkinson's disease severity (e.g., Hoehn and Yahr stages) to ensure their relevance and reliability.

At S640, the extracted features from the preprocessed ECG data are normalized. ECG signals may be normalized to standard amplitude and duration to facilitate consistent feature extraction and comparison across subjects. Additionally, the ECG signals may be segmented into individual cardiac cycles or fixed-length windows (e.g., 10-second ECG) to enable stage-specific and feature-based analysis. Signals longer than the fixed-length windows may be cropped, while shorter signals may be zero-padded.

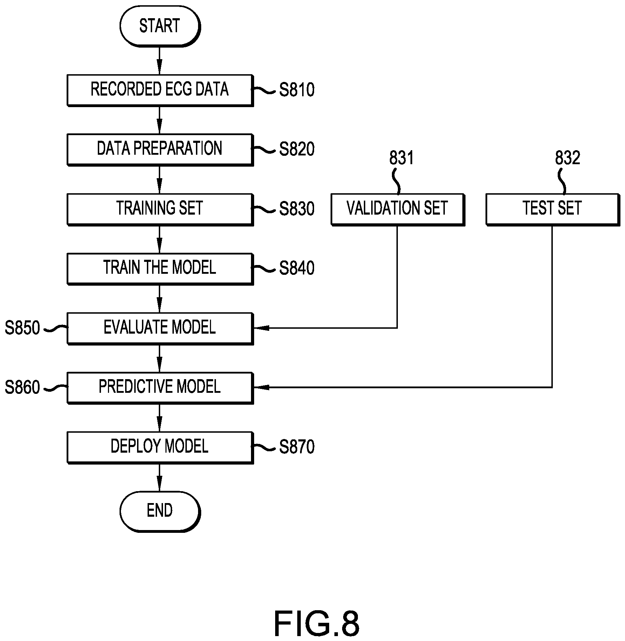

At S650, features are selected from the normalized features extracted from the preprocessed ECG data. For AI training described herein for Figs. 6, 7 and 8, selected features from the collected data may be labeled according to the corresponding H&Y stage for Parkinson's disease patients and as "normal" for control subjects. The labeling process should be consistent, accurate, and aligned with clinical evaluations conducted by qualified medical professionals. The labeling may also include demographic (e.g., age, sex) and clinical information (e.g., medication history, comorbidities) alongside ECG data to account for potential confounding factors during analysis. Feature selection at S650 and similar steps in Figs. 7 and 8 may be performed to identify the most relevant ECG-derived features for classification, reducing dimensionality and improving model performance. Model performance may be evaluated using metrics such as accuracy, precision, recall, F1 score, and confusion matrix to assess classification quality for each H&Y stage. Examples of feature selection techniques include mutual information, recursive feature elimination (RFE), and LASSO regression. Dimensionality reduction techniques include principal component analysis (PCA) or t-SNE, and may further optimize the feature set by retaining the most informative components while minimizing redundancy.

At S660, dimensions of the selected features may be reduced to improve performance. To enhance the accuracy of classifying Parkinson's Disease (Parkinson's disease) severity and progression, dimensionality reduction methods may be employed. These include both linear approaches, such as principal component analysis (PCA), and nonlinear techniques, often referred to as manifold learning, such as multidimensional scaling (MDS), local linear embedding (LLE), isomap, and t-distributed stochastic neighbor embedding (t-SNE). The features derived from ECG data are subsequently utilized as input to any of a variety of machine learning classifiers. Classifier models and algorithms that may be developed and implemented include traditional machine learning models such as logistic regression, random forests, support vector machines (SVMs), k-nearest neighbors (k-NN), as well as advanced algorithms such as gradient boosting methods (e.g., XGBoost, LightGBM, CatBoost), ensemble models, and deep neural networks.

Machine learning classifiers that may include multi-layer perceptron (MLP) networks, k-nearest neighbors (kNN), support vector machines with radial basis function kernels (RBF SVM), Gaussian naive Bayes, Gaussian process models, decision trees, random forests, stochastic gradient descent (SGD), and quadratic discriminant analysis (QDA). Furthermore, ECG data can be processed using advanced architectures such as deep residual neural networks (ResNet) and Transformer models. These models are trained with hyperparameters optimized through reinforcement learning strategies, improving their ability to predict Parkinson's disease severity and track disease progression effectively. Model parameters may be optimized through techniques such as grid search, random search, or Bayesian optimization for enhanced accuracy and generalizability.

At S670, a model is trained, tested and deployed based on the selected features. Several different types of machine learning models may be trained, tested, and deployed at S670. One example type is a classification algorithm. Examples of classification algorithms include support vector machines, random forests, or deep learning networks. Another example type is a regression model. Examples of regression models include linear regression models, gradient boosting models, or recurrent neural networks models to estimate progression trajectories.

The method of Fig. 6 may be implemented by multiple instances of the system 200 using the cloud 299 to train, test and deploy an artificial intelligence model. Instances of the system 200 may each be configured to obtain ECG data from different patients among a plurality of patients diagnosed with a neurological disease across a spectrum of stages, and from a plurality of individuals without the neurological disease. The ECG data may be labeled according to the corresponding severity stage for patients and as normal for individuals without the neurological disease in the preprocessing at S620. ECG features associated with characteristics of the neurological disease may be extracted at S630 from the labeled ECG data. The detected characteristics may comprise electrophysiological markers such as at least one of heart-rate variability (HRV) metrics, QT interval, QRS duration, and frequency-domain features.

The extracted ECG features and the ECG data may be processed in the normalization at S640 to identify patterns related to disease severity, and the features selected at S650 may be selected from among the identified patterns. A classifier model may be developed at S670 as the artificial intelligence model to classify the ECG data based on the ECG features and the identified patterns. The output of deploying the model may include progression metrics, including rate of change and projected future severity.

Fig. 7 illustrates a method for predicting and classifying Parkinson's disease severity and progression from ECG, in accordance with a representative embodiment.

The method of Fig. 7 may be performed by the system 200 including the controller 250.

Fig. 7 shows a flowchart illustrating the procedure for preparing data to train, test, and deploy an end-to-end deep learning (DL) model aimed at accurately classifying Parkinson's Disease (PD) severity into the five Hoehn and Yahr (H&Y) stages and monitoring disease progression in patients using multi-lead ECG data.

Fig. 7 illustrates the process for preparing data to train, test, and deploy an end-to-end deep learning (DL) model aimed at accurately classifying and monitoring the progression of Parkinson's disease (PD) in patients.

At S710, ECG data is obtained. ECG data may be acquired by the system 200. While the system 200 includes a 12-lead ECG, configurations ranging from single-lead to 2-, 3-, 4-, 5-, 12-, 15-, 16-, 22-, 80-lead, and beyond may also be utilized.

At S720, the ECG data is preprocessed. A preprocessing module of software executed by the controller 250 may comprise one or more algorithms for cleaning and preprocessing ECG signals to remove unwanted artifacts and noise such as power line interference and baseline wander, and to extract relevant features. Preprocessing steps at S720 may include noise removal.

At S730, the preprocessed ECG data is normalized. In this approach, multi-lead ECG data is normalized to ensure consistency and compatibility with the deep learning model.

At S740, the normalized ECG data is augmented for training. For example, data augmentation in this field is explained in An, J., Gregg, R. E., & Borhani, S., (2022) 44th Annual International Conference of the IEEE Engineering in Medicine & Biology Society (EMBC) (pp. 1283-1287). IEEE, "Effective data augmentation, filters, and automation techniques for automatic 12-lead ECG classification using deep residual neural networks," (July 2022), the contents of which are incorporated by reference herein in the entirety. To enhance model generalization during training, at S750 a range of data augmentation techniques may be used at S740. These include methods such as signal amplification, applying band-pass or high-pass filters, introducing baseline shifts or wander, adding noise from electromyographic (EMG) signals, and using techniques such as Flow-Mixup, Gaussian blur, or noise. Other augmentation methods involve random cropping, flipping, rotation, translation, and altering channel sequences through channel shuffling or reversal. Additionally, techniques such as Cutout, dropout, Mixup, CutMix, and adversarial training can be applied. Automated tools such as RandAugment, AutoAugment, AugLy, AutoFlip, and DeepAugment can streamline the implementation of these strategies. By creating more varied training data, these methods improve the model's robustness and its ability to generalize effectively.

An advanced deep learning architecture tailored for direct ECG signal analysis may be designed. For example, end-to-end deep residual neural networks in this field are explained in An, J., Bailey, B., & Gregg, R. E. (2023) Computing in Cardiology (CinC) (Vol. 50, pp. 1-4). IEEE, "Paradigm Shift from Feature-Based Machine Learning to End-to-End Deep Residual Neural Networks for Pediatric Age Classification from 12-Lead ECG," (October 2023), and An, J., Gregg, R. E., Bailey, B., Zhang, Y. H., & Dzikowicz, D. J. (2024) Computing in Cardiology (CinC) (Vol. 51). IEEE, "Left Bundle Branch Block Detection in 12-Lead ECG Using End-to-End Deep Learning with Explainability" (September 2024), the contents of which are incorporated by reference herein in the entirety.

Such architectures include:
- Convolutional Neural Networks (CNNs), such as Residual Neural Network (ResNet) or others, for feature extraction and spatial pattern recognition in ECG waveforms.
- Recurrent Neural Networks (RNNs) and their variants (e.g., Long Short-Term Memory [LSTM], Gated Recurrent Units [GRUs]) for capturing temporal dependencies in sequential ECG data.
- Hybrid Models combining CNNs and RNNs to leverage both spatial and temporal information.
- Transformer-Based Architectures (e.g., time-series transformers) for efficient handling of long-duration ECG sequences.

In the case of neural networks, preprocessing layers may be included within the neural networks to automatically handle tasks such as noise reduction and normalization, reducing dependency on external preprocessing steps. Multi-lead ECG inputs (e.g., all 12 leads) may be used, and lead-specific information may be fused within the network architecture to capture spatial correlations across leads. Data augmentation techniques may be implemented, such as by adding simulated noise or varying amplitude and duration, to increase dataset diversity and improve model robustness. Transfer learning may be employed by pretraining models on large, publicly available ECG datasets and fine-tuning them on Parkinson's disease-specific data to address challenges of limited data. These models may be trained on raw or minimally preprocessed ECG data to perform:
- Classification Tasks: Categorizing Parkinson's Disease (Parkinson's disease) severity into Hoehn and Yahr stages (1-5).
- Regression Tasks: Predicting Parkinson's disease progression metrics over time.
- Detection Tasks: Identifying Parkinson's disease from normal (healthy) ECGs or other cardiac abnormalities or arrhythmias.

At S750, a model is trained, tested and deployed based on the ECG data. Several different types of machine learning models may be trained, tested and deployed at S750. One example type is a classification algorithm. Examples of classification algorithms include support vector machines, random forests, or deep learning networks, such as convolutional neural networks (CNNs) and recurrent neural networks (RNNs). Another example type is a regression model. Examples of regression models include linear regression models, gradient boosting models, or recurrent neural networks models to estimate progression trajectories. The output of deploying the model may include progression metrics, including rate of change and projected future severity. Regression models for progression assessment may be implemented to predict changes in Parkinson's disease severity over time and include:
- Linear regression for simple relationships.
- Support vector regression (SVR) for capturing non-linear patterns.
- Polynomial regression for modeling complex trends.
- Random forest regression for ensemble-based predictions.
- Gradient boosting regression (e.g., XGBoost, LightGBM, CatBoost) for improved accuracy with boosting techniques.
- Deep learning-based regression, including:
   - Long short-term memory networks (LSTMs) for learning temporal dependencies.
   - Gated Recurrent Units (GRUs) for efficient sequence modeling.
   - Temporal Convolutional Networks (TCNs) for capturing long-range dependencies in time-series data.
   - Transformer-based models (e.g., Time-Series Transformers, BERT etc.) for advanced sequential modeling and feature extraction.
   - Bayesian regression for probabilistic modeling of progression uncertainty.
   - Gaussian Process Regression (GPR) for non-parametric modeling of disease progression.
   - Others.

Longitudinal ECG data may be paired with clinical Parkinson's disease assessments over time, to train and validate these models. Time-series analysis techniques such as autoregressive integrated moving average (ARIMA) or recurrent neural networks (RNNs), may be incorporated to account for temporal trends in Parkinson's disease progression.

Progression-specific features that may be detected from ECG data include:
- Temporal changes in RR, QTc intervals, or heart-rate variability (HRV) metrics.
- Rate of decline in electrophysiological parameters indicative of autonomic dysfunction.
- Dynamic ECG patterns observed during specific tests (e.g., postural changes or stress tests).

Regularization techniques such as Lasso and Ridge may be used to improve model generalizability and reduce overfitting. Models may be validated using metrics such as mean absolute error (MAE), root mean square error (RMSE), and R² to ensure accurate prediction of Parkinson's disease progression. Model validation and testing may include statistical analysis to compare model performance across validation and test datasets, ensuring consistency and identifying potential biases. Robustness testing under real-world conditions may be implemented such as varying signal quality (e.g., noisy or incomplete ECG data) or differences in patient demographics, comorbidities, or ECG acquisition protocols. Ablation studies may be performed to understand the contribution of various features, leads, or components of the model to its overall performance. Interpretability may be validated by comparing model predictions with clinical expertise, using explainability tools such as Gradient-weighted Class Activation Mapping (Grad-CAM), Shapley Additive Explanations (SHAP), Local Interpretable Model-agnostic Explanations (LIME), or other methods. Thresholds may be established for clinical acceptability, ensuring the model meets performance standards for deployment in healthcare settings. Independent "gold standard" datasets annotated by clinical experts may be used for final benchmarking before deployment.

The training at S750 involves feeding the normalized data into the model, which learns to map the input data to the desired output without the need for explicit feature extraction. Unlike traditional machine learning methods, which rely on predefined features, this end-to-end model automatically identifies and extracts relevant patterns from the raw ECG data. Through this automated learning process, the model optimizes its internal representations, improving its ability to accurately classify Parkinson's disease severity and track disease progression over time. Model training may be optimized with techniques such as: batch normalization and dropout for regularization, and/or learning rate scheduling and adaptive optimizers such as Adam, RMSprop, or others.

A variety of deep learning models can be applied to this task, such as Long Short-Term Memory (LSTM) networks, Convolutional Neural Networks (CNNs), and popular architectures such as Alex Neural Network (AlexNet), SqueezeNet, Google LeNet (GoogLeNet), ShuffleNet, MobileNet, Visual Geometry Group Network (VGG), Residual Neural Network (ResNet), Squeeze-and-Excitation Residual Network (SE-ResNet), Next Generation Residual Networks (ResNeXt), Densely Connected Convolutional Network (DenseNet), Squeeze-and-Excitation Networks (SENet), GoogLeNet Inception Network (Inception), Neural Architecture Search Network (NASNet), Transformer networks, Capsule Networks, attention-based models, Gated Recurrent Unit (GRU), Bidirectional Encoder Representations from Transformers (BERT) for time-series, U-shaped Convolutional Network for segmentation (UNet), and Deep Belief Networks (DBNs). These models are particularly well-suited for capturing temporal dependencies, spatial features, and complex interactions within multi-lead ECG data. As a result, these deep learning models enhance performance in various areas such as early diagnosis, severity classification, monitoring the progression of Parkinson's disease, and assessing the disease over time.

Fig. 8 illustrates a method for predicting and classifying Parkinson's disease severity and progression from ECG, in accordance with a representative embodiment.

The method of Fig. 8 may be performed by the system 200 including the controller 250.

Fig. 8 illustrates a comprehensive flowchart outlining the end-to-end process for training, testing, and deploying both feature-based machine learning (ML) models and end-to-end deep learning (DL) models for the reliable detection, classification, and monitoring of Parkinson's Disease (PD) using multi-lead ECG data.

Fig. 8 shows a flowchart depicting the process of training, testing, and deploying feature-based machine learning (ML) and end-to-end deep learning (DL) models for reliably classifying Parkinson's Disease (PD) severity into the five Hoehn and Yahr (H&Y) stages and monitoring disease progression in patients using multi-lead ECG data. Modeling may be personalized to predict individual disease trajectories by leveraging patient-specific data, such as baseline ECG parameters, demographics, and comorbidities.

At S810, ECG data is recorded to begin the workflow.

At S820, data is prepared. Data preparation at S820 may include extraction of relevant features that capture the characteristics of the cardiac signals. This raw data is then pre-processed to ensure the raw data is clean, consistent and properly formatted for subsequent analysis and model training.

At S830, a training set is developed as labeled data which includes information on Parkinson's disease severity, progression, and patient status.

At S840, a model is trained. The labeled data enables the model to learn the relationships between ECG patterns and clinical outcomes associated with Parkinson's disease.

At S850, the model is evaluated using a validation set 831. A model may be tested on an independent validation dataset, ideally from a different cohort, to verify generalizability. In the evaluation stage, a trained model is evaluated on unseen data to assess the trained model's generalization capabilities and real-world performance. Rigorous cross-validation techniques may be used to assess model performance and ensure robust model training and prevent overfitting. Cross-validation techniques include K-Fold cross-validation that splits the dataset into k folds to iteratively train and test the model on different subsets, ensuring robust performance metrics, or stratified cross-validation that maintains the distribution of Parkinson's disease severity stages (or other relevant classes) across folds to avoid biased performance estimates. Also, or alternatively, the model may be tested on independent datasets to evaluate generalizability, including: external validation datasets using data from different populations, devices, or institutions to confirm model robustness. Models may be evaluated on external datasets or real-world clinical data to assess robustness and applicability across diverse populations. Temporal validation may be used to assess performance on data collected at different time points to evaluate model reliability over time.

At S860, a predictive model is developed and applied to a test set 832. Various performance metrics are used during testing, such as accuracy, sensitivity, specificity, robustness, F1 score, area under the receiver operating characteristic curve (AUC-ROC), area under the precision-recall curve (AUC-PR), Matthew's Correlation Coefficient (MCC), and others, all of which provide a comprehensive understanding of the model's effectiveness in detecting, classifying, and diagnosing Parkinson's disease. Evaluation metrics tailored to the task may be applied. For example, for classification and detection tasks, accuracy, precision, recall, F1-score, confusion matrix, and AUC-ROC may be used as metrics. For regression tasks, mean absolute error (MAE), root mean square error (RMSE), mean squared error (MSE), and R² may be used as metrics. Metrics may be evaluated to identify contributions to the accuracy of Hoehn and Yahr stage predictions.

At S870, the model is deployed. The output of deploying the model may include progression metrics, including rate of change and projected future severity. Deployed models described herein may be subject to explainability techniques such as Grad-CAM, SHAP or LIME to understand model decision-making and ensure clinical interpretability of results. These types of explainability tools may be incorporated to enable visualization as to how a model interprets ECG signals and to ensure clinical interpretability.

Deployment and integration of models in the methods described herein may involve development of a user-friendly interface for the user interface 254 or the display 280. The development may involve creating an intuitive graphical user interface (GUI) for clinicians, enabling them to: input ECG data seamlessly from standard devices such as, for example, electrocardiographs or portable ECG monitors, view Parkinson's disease severity classifications such as Hoehn and Yahr stages and progression assessments with clear visualizations and explanations, access historical patient data for tracking disease progression over time, and ensure compatibility with various input formats such as digital ECG files like Digital Imaging and Communications in Medicine (DICOM), Extensible Markup Language (XML), Waveform Database (WFDB), or raw waveform data. Real-time clinical deployment may involve integrating the new technologies in system 200 with existing clinical workflows, such as electronic health record (EHR) systems, to ensure smooth adoption. Real-time analysis of ECG data during patient visits or remote monitoring sessions is enabled to provide outputs in a clinically interpretable format, including: numerical scores such as stage-specific risk probabilities, or annotated ECG waveforms highlighting features linked to Parkinson's disease-related abnormalities. The deployed technology may support multi-lead and single-lead ECG data for flexible use across various settings such as hospitals, outpatient clinics, and home care. The methods and system may be scalable and support deployment on platforms, such as: edge devices such as portable ECG or electrocardiograph/cardiograph monitors for real-time analysis in point-of-care settings, as well as cloud-based services that allow for centralized processing for large-scale implementation and remote monitoring. In some embodiments, deployment may be via hybrid systems that combine edge and cloud-based solutions for optimized performance.

Encryption and secure access protocols may be implemented to protect patient information. Use of the cloud 299 may enable compatibility across different operating systems and devices including electrocardiograph/cardiograph, desktops, tablets, and smartphones. Models may be maintained and updated by incorporating mechanisms for periodic updates to the underlying machine learning or deep learning models based on new data or clinical guidelines. Additionally, continuous learning systems may be enabled to refine performance using feedback from clinicians and real-world use.

The flowchart for Fig. 8 highlights the integration of feature-based machine learning approaches with deep learning models, enabling the development of a hybrid model that combines the strengths of both techniques. This hybrid approach allows the system to benefit from the interpretability and efficiency of traditional machine learning while leveraging the advanced pattern recognition capabilities of deep learning models. The combination of these methodologies ensures the development of a scalable, flexible, and highly accurate solution for Parkinson's disease detection, classification, and monitoring. This integrated framework is designed to be adaptable to a variety of clinical settings, offering enhanced diagnostic accuracy and supporting timely interventions, ultimately improving patient outcomes and care quality.

Fig. 9 illustrates progressions and progressions for predicting and classifying Parkinson's disease severity and progression from ECG, in accordance with a representative embodiment.

In Fig. 9, a user interface 981 shows an initial projection of future severity 3P from an initial session, an actual severity 3A which is comparable to the future severity 3P once the severity is measured at time #3, and a subsequent of future severity 4P from a subsequent session. Fig. 9 illustrates how projections of severity may be updated based on objective analysis by an artificial intelligence model. The use of cloud 299 enables collection and analysis of ECG data from a multitude of patients over time so that the severity classifications and projections reflect evidence from a wide variety of sources.

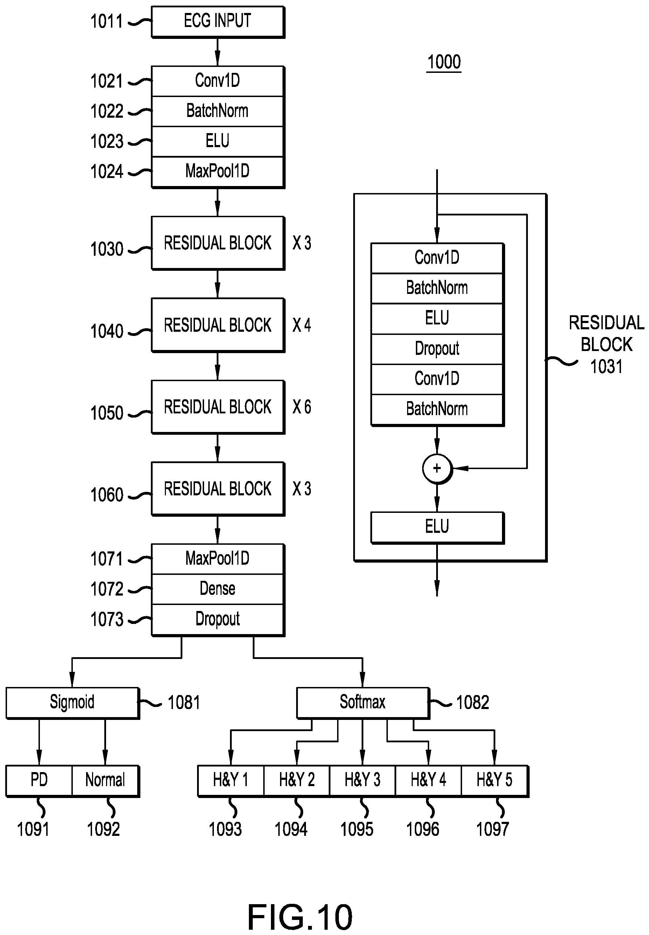

Fig. 10 illustrates a deep learning (DL) model for predicting and classifying Parkinson's disease severity and progression from ECG, in accordance with a representative embodiment.

Fig. 10 shows an example architecture of an end-to-end deep learning (DL) model using a residual network (ResNet) to detect Parkinson's Disease (PD) from normal ECG signals and classify PD severity into the five Hoehn and Yahr (H&Y) stages.

Fig. 10 illustrates an example architecture of an end-to-end deep learning (DL) model based on the residual network (ResNet), specifically designed to leverage the power of DL for detecting Parkinson's Disease (PD) from normal ECG and classifying PD severity into the five Hoehn and Yahr (H&Y) stages. The ResNet model takes multi-lead ECG signals as inputs at 1011. The structure of the ResNet model includes a one-dimensional (1D) convolutional layer 1021 followed by a first residual block 1030, a second residual block 1040, a third residual block 1050, and a fifth residual block 1060. After the one-dimensional (1D) convolutional layer 1021, batch normalization (BatchNorm) 1022 is applied, followed by the exponential linear unit (ELU) activation function 1023 with an alpha parameter set to 1.0, dropout with a probability of 0.2, and adaptive max-pooling 1024 (MaxPool1D). The output layer 1071 of the ResNet model is designed for both binary classification (Parkinson's disease detection) and multiclass classification (Parkinson's disease severity into the 5 H&Y stages). The output layer 1071 feeds into a fully connected (dense) layer 1072 with a dropout probability of 0.2, using a sigmoid activation function 1081 for Parkinson's disease detection and a softmax activation function 1082 for Parkinson's disease severity classification. The sigmoid activation function 1081 produces probabilities ranging from 0 to 1 for detecting Parkinson's disease, while the softmax activation function 1082 classifies the Parkinson's disease severity into one of the five H&Y stages (1-5). The optimal threshold for detecting Parkinson's disease from probabilities can be determined using various methods, such as setting a default threshold of 0.5, optimizing based on the Receiver Operating Characteristic (ROC) curve by maximizing Youden's Index, using the Precision-Recall (PR) curve to find the threshold that maximizes the F1-score, or employing cost-sensitive thresholding to adjust for clinical priorities, such as prioritizing sensitivity to reduce false negatives or specificity to minimize false positives. The final threshold selection should be validated on a separate test set to ensure robustness and clinical applicability. The ResNet model is trained to minimize a loss function using cross-entropy loss for Parkinson's disease detection and categorical cross-entropy loss for Parkinson's disease severity classification into the five H&Y stages. This architecture enables accurate detection of Parkinson's disease and effective classification of its severity.

An example residual block 1031 is shown on the right side and includes a Conv1D layer, a BatchNorm layer, an ELU layer, a dropout layer, another Conv1D layer, a BatchNorm layer, and another ELU layer.

Fig. 11 illustrates a computer system, on which a method for predicting and classifying Parkinson's disease severity and progression from ECG is implemented, in accordance with another representative embodiment.

Referring to Fig. 11, the computer system 1100 includes a set of software instructions that can be executed to cause the computer system 1100 to perform any of the methods or computer-based functions disclosed herein. The computer system 1100 may operate as a standalone device or may be connected, for example, using a network 1101, to other computer systems or peripheral devices. In embodiments, a computer system 1100 performs logical processing based on digital signals received via an analog-to-digital converter.

In a networked deployment, the computer system 1100 operates in the capacity of a server or as a client user computer in a server-client user network environment, or as a peer computer system in a peer-to-peer (or distributed) network environment. The computer system 1100 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 1100 can be incorporated as or in a device that in turn is in an integrated system that includes additional devices. In an embodiment, the computer system 1100 can be implemented using electronic devices that provide voice, video or data communication. Further, while the computer system 1100 is illustrated in the singular, the term "system" shall also be taken to include any collection of systems or sub-systems that individually or jointly execute a set, or multiple sets, of software instructions to perform one or more computer functions.

As illustrated in Fig. 11, the computer system 1100 includes a processor 1110. The processor 1110 may be considered a representative example of a processor of a controller and executes instructions to implement some or all aspects of methods and processes described herein. The processor 1110 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 1110 is an article of manufacture and/or a machine component. The processor 1110 is configured to execute software instructions to perform functions as described in the various embodiments herein. The processor 1110 may be a general-purpose processor or may be part of an application specific integrated circuit (ASIC). The processor 1110 may also be a microprocessor, a microcomputer, a processor chip, a controller, a microcontroller, a digital signal processor (DSP), a state machine, or a programmable logic device. The processor 1110 may also be a logical circuit, including a programmable gate array (PGA), such as a field programmable gate array (FPGA), or another type of circuit that includes discrete gate and/or transistor logic. The processor 1110 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 1100 further includes a main memory 1120 and a static memory 1130, where memories in the computer system 1100 communicate with each other and the processor 1110 via a bus 1108. Either or both of the main memory 1120 and the static memory 1130 may be considered representative examples of a memory of a controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 1120 and the static memory 1130 are articles of manufacture and/or machine components. The main memory 1120 and the static memory 1130 are computer-readable mediums from which data and executable software instructions can be read by a computer (e.g., the processor 1110). Each of the main memory 1120 and the static memory 1130 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 1100 further includes a video display unit 1150, such as a liquid crystal display (LCD), an organic light emitting diode (OLED), a flat panel display, a solid-state display, or a cathode ray tube (CRT), for example. Additionally, the computer system 1100 includes an input device 1160, such as a keyboard/virtual keyboard or touch-sensitive input screen or speech input with speech recognition, and a cursor control device 1170, such as a mouse or touch-sensitive input screen or pad. The computer system 1100 also optionally includes a disk drive unit 1180, a signal generation device 1190, such as a speaker or remote control, and/or a network interface device 1140.

In an embodiment, as depicted in Fig. 11, the disk drive unit 1180 includes a computer-readable medium 1182 in which one or more sets of software instructions 1184 (software) are embedded. The sets of software instructions 1184 are read from the computer-readable medium 1182 to be executed by the processor 1110. Further, the software instructions 1184, when executed by the processor 1110, perform one or more steps of the methods and processes as described herein. In an embodiment, the software instructions 1184 reside all or in part within the main memory 1120, the static memory 1130 and/or the processor 1110 during execution by the computer system 1100. Further, the computer-readable medium 1182 may include software instructions 1184 or receive and execute software instructions 1184 responsive to a propagated signal, so that a device connected to a network 1101 communicates voice, video or data over the network 1101. The software instructions 1184 may be transmitted or received over the network 1101 via the network interface device 1140.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, predicting and classifying Parkinson's disease severity and progression from ECG enables classification and tracking of the severity of Parkinson's disease and other neurological diseases using multi-lead electrocardiograms (ECGs). The methods and system described above provide an objective, continuous, and easily accessible approach to PD monitoring. By leveraging machine learning or deep learning algorithms applied to ECG data, the methods and system can classify PD severity into stages and monitor disease progression over time such as daily, monthly, or yearly. The methods and system facilitate improved disease management, support the development of personalized treatment strategies, and enhance the design and execution of clinical trials. By addressing the limitations of traditional clinical assessments, this method fulfills a critical need for frequent, reliable, and objective PD monitoring. The methods and system enhance patient care through frequent, objective assessments derived from ECG data and also support research and clinical trials by providing robust, data-driven insights into disease progression and its correlation with electrophysiological markers.

Although predicting and classifying Parkinson's disease severity and progression from ECG has been described with reference to several exemplary embodiments, it is understood that the words that have been used are words of description and illustration, rather than words of limitation. Changes may be made within the purview of the appended claims, as presently stated and as amended, without departing from the scope and spirit of predicting and classifying Parkinson's disease severity and progression from ECG in its aspects. Although predicting and classifying Parkinson's disease severity and progression from ECG has been described with reference to particular means, materials and embodiments, predicting and classifying Parkinson's disease severity and progression from ECG is not intended to be limited to the particulars disclosed; rather predicting and classifying Parkinson's disease severity and progression from ECG extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for classifying and projecting neurological disease severity based on electrocardiograph (ECG), comprising:
a controller (250) comprising a memory (251) that stores instructions and a processor (252) that executes the instructions, wherein, when executed by the processor, the instructions cause the system to:
obtain (S505) initial ECG data;
detect (S515) characteristics indicative of a neurological disease from the initial ECG data;
classify (S520) severity of the neurological disease based on the detected characteristics from the initial ECG data; and
predict (S525) a future severity of the neurological disease based on the classified severity.

2. The system of claim **1,** wherein, when executed by the processor, the instructions cause the system further to:
obtain (S550) subsequent ECG data;
detect (S555) characteristics of the neurological disease from the subsequent ECG data;
classify (S560) severity of the neurological disease based on the detected characteristics from the subsequent ECG data; and
update (S565) the predicted future severity of the neurological disease to generate (S570) a disease progression based on the classified severity from both the initial ECG data and subsequent ECG data.

3. The system of claim **1,** wherein the neurological disease is Parkinson's disease.

4. The system of claim **1,** wherein, when executed by the processor, the instructions cause the system further to:
provide the initial ECG data as input to an artificial intelligence model to detect the characteristics indicative of the neurological disease.

5. The system of claim **1,** wherein, when executed by the processor, the instructions cause the system further to:
track (S570) a progression of the neurological disease over time by analyzing the classified severity from the initial ECG data and at least one previous ECG dataset.

6. The system of claim 2, further comprising:
a display (280), wherein, when executed by the processor, the instructions cause the system further to:
visually present (S575) the predicted progression of disease severity over multiple ECG datasets.

7. The system of claim 1, wherein the characteristics of the neurological disease comprise electrophysiological markers.

8. The system of claim 7, wherein the electrophysiological markers comprise at least one of heart-rate variability (HRV) metrics, QT interval, QRS duration, and frequency-domain features.

9. The system of claim 4, wherein the artificial intelligence model comprises at least one of a classification algorithm or a regression model.

10. The system of claim 1, wherein, when executed by the processor, the instructions cause the system further to:
generate and output a severity stage of the neurological disease along with a confidence score for the severity stage.

11. The system of claim 1, wherein, when executed by the processor, the instructions cause the system further to:
store the detected characteristics, classified severity, and predicted progression of the neurological disease in a secured cloud-based repository (299), a server (200), or an electrocardiograph device (200).

12. The system of claim 1, wherein, when executed by the processor, the instructions cause the system further to:
select an artificial intelligence model from among a plurality of available artificial intelligence models based on predefined selection criteria; and
apply the selected artificial intelligence model to the initial ECG data to detect the characteristics of the neurological disease.

13. The system of claim 12, wherein, when executed by the processor, the instructions cause the system further to:
initiate a first mode to detect the characteristics of the neurological disease, including selecting the artificial intelligence model to apply the selected artificial intelligence model to the initial ECG data; and
initiate a second mode to track progression of known cases of the neurological disease over time across a plurality of patients.

14. The system of claim 12, wherein the system is implemented on a mobile electronic device.

15. A method for training an artificial intelligence model to classify neurological disease severity based on electrocardiograph (ECG) data, the method comprising:
obtaining (S610) ECG data from a plurality of patients diagnosed with a neurological disease across a spectrum of stages, and from a plurality of individuals without the neurological disease;
labeling (S630) the ECG data according to the corresponding severity stage for patients and as normal for individuals without the neurological disease;
extracting (S630) ECG features associated with characteristics of the neurological disease from the labeled ECG data;
processing (S670) the extracted ECG features and the ECG data to identify patterns related to disease severity; and
developing (S670) a classifier model as the artificial intelligence model to classify the ECG data based on the ECG features and the identified patterns
